(19) Europäisches Patentamt
European Patent Office
Office européen des brevets

(11) Veröffentlichungsnummer: **0 510 596 A2**

(12) **EUROPÄISCHE PATENTANMELDUNG**

(21) Anmeldenummer: **92106858.1**

(51) Int. Cl.5: **C07C 59/135**, C07C 51/04

(22) Anmeldetag: **22.04.92**

(30) Priorität: **26.04.91 DE 4113620**

(43) Veröffentlichungstag der Anmeldung:
**28.10.92 Patentblatt 92/44**

(84) Benannte Vertragsstaaten:
**DE FR GB IT NL**

(71) Anmelder: **HOECHST AKTIENGESELLSCHAFT**
**Postfach 80 03 20**
**W-6230 Frankfurt am Main 80(DE)**

(72) Erfinder: **Gries, Thomas, Dr.**
**Johannesallee 18**
**W-6230 Frankfurt am Main 80(DE)**
Erfinder: **Schwertfeger, Werner, Dr.**
**Erlenstrasse 8**
**W-6306 Langgöns(DE)**
Erfinder: **Kottmann, Hariolf, Dr.**
**Am Alten Birnbaum 6**
**W-6238 Hofheim am Taunus(DE)**
Erfinder: **Ebmeyer, Frank, Dr.**
**Palleskestrasse 29**
**W-6230 Frankfurt am Main 80(DE)**

(54) **Verfahren zur Herstellung von Perfluorethercarbonsäuren.**

(57) Die Erfindung betrifft ein Verfahren zur Herstellung von Perfluorethercarbonsäuren der Formel (I) $R_f$-COOH aus Perfluoretheracylfluoriden der Formel (II):

(II) $R_f$-COF

mit $R_f$ gleich $C_mF_{2m+1}$-O-$(C_3F_6O)_n$-CF$(CF_3)$-
worin m = 1 - 8 und n = 0 - 8 ist,
bei dem man die Acylfluoride (II) mit der 0,5- bis 5-fachen Gewichtsmenge eines fluorierten inerten Lösungsmittels verdünnt und dann mit 2 - 15 Mol Wasser pro Mol Acylfluorid intensiv vermischt und die nach Phasentrennung erhaltene organische Phase destillativ vom Lösungsmittel befreit.

Fig. 1

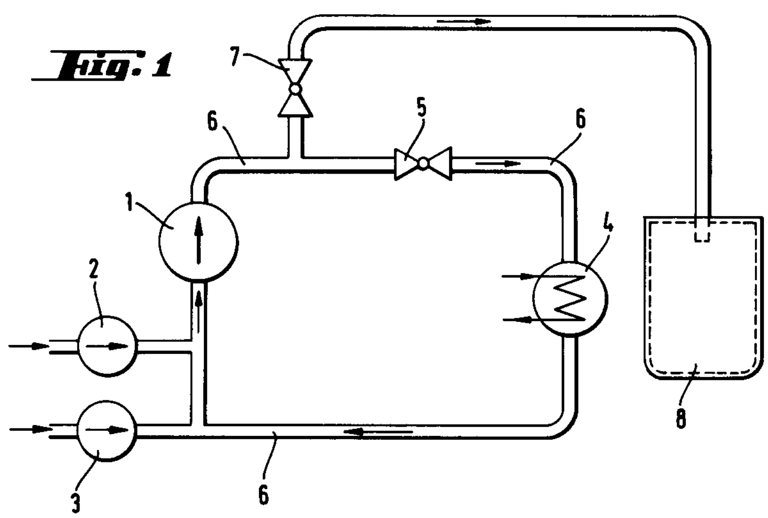


EP 0 510 596 A2

Die Erfindung betrifft ein Verfahren zur Herstellung von Perfluorethercarbonsäuren der Formel (I) $R_f$-COOH aus Perfluoretheracylfluoriden der Formel (II):

(II) $R_f$-COF

mit $R_f$ gleich $C_mF_{2m+1}$-O-$(C_3F_6O)_n$-CF($CF_3$)-,
worin m = 1 - 8 und n = 0 - 8 ist.

Perfluorethercarbonsäuren sind wichtige Zwischenprodukte für die Herstellung von nicht brennbaren, temperaturstabilen Perfluorpolyethern, die als Inertflüssigkeiten dienen (US-PS 3 665 041, US-PS 3 250 808). Sie werden außerdem als Tenside und Hydrophobierungsmittel eingesetzt.

Die Herstellung von Perfluoralkansäuren durch Elektrofluorierung von Acylfluoriden und anschließende Hydrolyse der gebildeten Perfluoralkancarbonsäurefluoride ist bereits bekannt (US-PS 4 022 824, US-PS 4 927 962).

Es ist auch bereits bekannt, Perflourethercarbonsäuren durch Oligomerisierung perfluorierter Epoxide und Hydrolyse der dabei entstandenen Perfluoretheracylfluoride herzustellen (US-PS 3 274 239, US-PS 3 250 808). In der US-PS 3 274 239 wird jedoch nicht näher angegeben, auf welche Weise die Hydrolyse durchgeführt werden soll. Nach den Beispielen 7 und 9 der US-PS 3 250 808 werden dazu Perfluoretheracylfluoride in Perfluordimethylcyclobutan gelöst und mit einem 60-fachem Wasserüberschuß hydrolysiert. Die genannten Beispiele zeigen, daß dieses Verfahren niedrige Ausbeuten von 65 bzw. 25 % ergibt und daß verhältnismäßig große Wasser- und Lösungsmittelmengen sowie lange Reaktionszeiten (60 min bis 60 h) benötigt werden. Das Verfahren ist deshalb für eine technische Anwendung wenig geeignet.

Nicht erwähnt worden ist, daß die so erhaltenen Perfluorethercarbonsäuren Fluoridionen enthalten (da bei der Reaktion wäßrige Flußsäure entsteht), die zur Korrosion der anschließend benutzten Destillationsapparatur führen.

Es wurde nun ein Verfahren gefunden, das es ermöglicht, auf einfache Weise - und somit auch im technischen Maßstab - fluoridarme, nebenproduktfreie Perfluorethercarbonsäuren aus den Perfluoretheracylfluoriden zu gewinnen.

Gegenstand der Erfindung ist ein Verfahren zur Herstellung von Perfluorethercarbonsäuren der Formel (I) $R_f$-COOH aus Perfluoretheracylfluoriden der Formel (II):

(II) $R_f$-COF

mit $R_f$ gleich $C_mF_{2m+1}$-O-$(C_3F_6O)_n$-CF($CF_3$)-
worin m = 1 - 8 und n = 0 - 8 ist,

dadurch gekennzeichnet, daß man die Acylfluoride (II) mit der 0,5- bis 5-fachen Gewichtsmenge eines fluorierten inerten Lösungsmittels verdünnt und dann mit 2 - 15 Mol Wasser pro Mol Acylfluorid intensiv vermischt und die nach Phasentrennung erhaltene organische Phase destillativ vom Lösungsmittel befreit.

Das Perfluoretheracylfluorid wird dabei zur entsprechenden Säure (I) hydrolysiert:

$R_fCOF + H_2O \dashrightarrow R_fCOOH + HF$ aq.

Man erhält danach zwei Phasen: eine leichte wäßrige Phase, die aus einer verdünnten wäßrigen Flußsäure besteht, und eine schwere organische Phase, die aus Perfluorethercarbonsäure und Lösungsmittel besteht. Durch einfache Phasentrennung wird die organische Phase abgetrennt. Der Fluoridgehalt der organischen Phase ist abhängig vom Molekulargewicht der Säure (I); er liegt zwischen 10 und 500 ppm. Liegt er über 100 ppm, so ist es von Vorteil, ihn durch eine einfache wäßrige Extraktion der organischen Phase auf einen Wert unter 100 ppm zu reduzieren.

Dieser Wert ist niedrig genug, um in gebräuchlichen Edelstahlapparaturen eine korrosionsfreie Destillation der organischen Phase zur Trennung von Lösungsmittel und Perfluorethercarbonsäure (I) zu ermöglichen.

Durch Destillation der organischen Phase, ggf. nach Extraktion, wird die Säure in einer Reinheit > 99 % und praktisch fluoridfrei erhalten. Der Fluoridgehalt ist im allgemeinen < 10 ppm; nur bei niedrigem Molekulargewicht der Carbonsäure liegt er zwischen 10 und 30 ppm.

Wurde ein einziges Säurefluorid (II) zur Hydrolyse eingesetzt, dann kann auf eine Destillation der so erhaltenen Säure verzichtet werden, da die Säure nach destillativer Abtrennung (vorzugsweise mit einem Dünnschichtverdampfer) des niedriger siedenden Lösungsmittels nur noch Wasser in einem Anteil zwischen 1 und 5 Gew.-% enthält. Dieser Wassergehalt ist für die meisten Anwendungen ohne Bedeutung. Wenn man jedoch ein Gemisch aus mehreren Säurefluoriden (II) hydrolysiert, so erhält man eine Lösung eines

Gemisches der entsprechenden Säuren (I), das nach Abdestillieren des Lösungsmittels noch destillativ in die einzelnen Säuren getrennt werden muß.

Als fluorierte inerte Lösungsmittel für das Verfahren sind geeignet:
1-H-Perfluorhexan $C_6F_{13}H$,
Perfluoralkane der Formel $C_aF_{2a+2}$ mit a = 5 - 12,
Chlorfluoralkane der Formel $C_2Cl_3F_3$ und $C_3Cl_2F_5H$,
Fluorierte Ether und Polyether der Formel

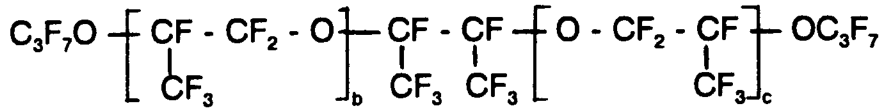

mit b = 0 - 4 und c = 0 - 4
oder

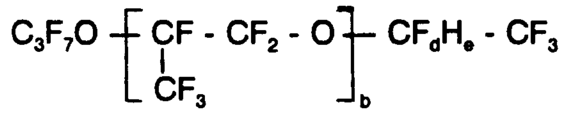

mit b = 0 - 4 und d = 1, e = 1 oder d = 2, e = 0
Die genannten fluorierten Alkane werden beschrieben in A. M. Lovelace, Aliphatic Fluorine Compounds, Reinhold Publ. Corp., New York, 1958, und die genannten fluorierten Ether und Polyether in DE-OS 3 828 848 und US-PS 3 250 808.

Besonders bevorzugt sind 1,1,2-Trichlor-1,2,2-trifluorethan, Perfluorhexan, Perfluorpropylethylether, Perfluorpropyltetrafluorethylether und 1-H-Perfluorhexan, vor allem aber der Chlorfluorkohlenwasserstoff F 113 (1,1,2-Trichlor-1,2,2-trifluorethan) wegen seines günstigen Siedepunktes von 47 °C und seines niedrigen Preises.

Vorzugsweise wird die 0,5- bis 2-fache Gewichtsmenge an Lösungsmittel, bezogen auf Perfluoretheracylfluorid (II) eingesetzt. Zur Hydrolyse werden vorzugsweise 9 bis 12 Mol Wasser pro Mol Perfluoretheracylfluorid eingesetzt. Diese Wassermenge ist um den Faktor 5 bis 7 niedriger als gemäß der oben erwähnten US-PS 3 250 808. Der bevorzugte etwa 9 - 12-fache Überschuß an Wasser über die stöchiometrische Menge hat sich als vorteilhaft erwiesen, um den entstehenden Fluorwasserstoff in Form einer etwa 10 %igen wäßrigen Flußsäure abzutrennen.

Für die oben erwähnte Fluoridextraktion der organischen Phase, die, falls nötig, vor der Abtrennung des Lösungsmittels durchgeführt wird, versetzt man die organische Phase aus der Hydrolyse - Lösungsmittel und Säure (I) - direkt mit der 0,1- bis 0,5-fachen Menge Wasser.
Der durch Extraktion der organischen Phase erhaltene wäßrige Extrakt enthält bis zu 1 Gew.- % Perfluorethercarbonsäuren (I) und wird bei kontinuierlicher Arbeitsweise vorteilhaft für die Hydrolyse der Perfluoretheracylfluoride (II) erneut eingesetzt.
Dadurch wird die Ausbeute erhöht und Rückstände werden vermieden. Die Gesamtausbeute an Säure ist dann größer als 97 %.

Die Herstellung der Perfluoretheracylfluoride kann durch Oligomerisierung von Hexafluorpropenoxid (HFPO) erfolgen (US-PS 3 247 239 und US-PS 3 250 808). Hierbei entsteht ein Gemisch oligomerer Säurefluoride (II), das ohne vorhergehende Trennung der Oligomeren in die erfindungsgemäße Hydrolyse eingesetzt werden kann.
Für die technische Ausführung der Hydrolyse und - falls durchgeführt - der Extraktion sind prinzipiell alle Apparate geeignet, die eine gute Durchmischung von organischer und wäßriger Phase bei der Hydrolyse ermöglichen, z. B. durch mechanische Rührer, durch eine Kreiselpumpe, durch Strömungsmischer oder durch Ultraschall.

Als vorteilhaft für eine diskontinuierliche Hydrolyse hat sich ein Rührkessel erwiesen; für ein kontinuierliches Verfahren sind ein Strömungsrohr mit Mischelementen oder ein ringförmiger Reaktor (Loop-Reaktor) mit einer schnelldrehenden Pumpe zur Vermischung gut geeignet. Die Extraktion kann in den gleichen Apparaten durchgeführt werden; bewährt haben sich aber auch ein Mixer-Settler und eine Schwingbodenkolonne.

Als Werkstoffe für die Apparate eignen sich besonders Kunststoffe, wie Polyethylen, Polypropylen, PVC, kunststoffausgekleideter Stahl und solche Metalle, die beständig gegen fluoridhaltige Säuren sind, z.

B. Hastelloy-Stähle. Für die Extraktionsapparatur kann auch Glas als Werkstoff verwendet werden.

Das beschriebene Verfahren besitzt folgende Vorteile:
Es ermöglicht kurze Reaktionszeiten und ergibt hohe Raum-Zeit-Ausbeuten von 0,5 kg/(h•l) im Rührkessel, 2,0 kg/(h•l) im Strömungsmischer und 30 kg/(h•l) im Pumpenmischer, verglichen mit 0,1 kg/(h•l) nach dem in der US-PS 3 250 808 beschriebenen Verfahren. Mit der bevorzugten niedrigen Lösungsmittelmenge (0,5- bis 2-fache Menge) und unter Rückführung von Lösungsmittel und ggf. Extraktionswasser werden Ausbeuten > 97 % und hohe Reinheiten erzielt.

Das Verfahren ermöglicht insbesondere die Herstellung hochsiedender, thermisch begrenzt stabiler Perfluorethercarbonsäuren in reiner Form, da nach der Hydrolyse des entsprechenden einzelnen Säurefluorids nur das niedrig siedende Lösungsmittel entfernt werden muß und keine Destillation der Säure erforderlich ist.

Eine besondere Ausführungsform des Verfahrens dient zur Umwandlung eines Gemisches von Perfluoretheracylfluoriden der Formel (II), worin m gleich 3 und n gleich 0, 1, 2 oder 3 ist, wie es durch katalytische Oligomerisierung von Hexafluorpropenoxid erhalten wird (US-PS 4 874 557), in die entsprechenden einzelnen Perfluorethercarbonsäuren. Durch Hydrolyse des genannten Säurefluoridgemisches in Trichlortrifluorethan und Fluoridextraktion (wie oben beschrieben) wird eine Lösung der entsprechenden Carbonsäuren (I) erhalten, die 50 bis 100 ppm Fluorid und 3 - 4 Gew.-% Wasser enthält.

Geringe feste oder hochsiedende Verunreinigungen aus dem Oligomerisierungskatalysator, wie z.B. Kaliumsalz und Adipinsäuredinitril, können bei der direkten Fraktionierung dieser Lösung durch Rektifikation im Vakuum dazu führen, daß ein Teil der Carbonsäuren zersetzt wird. Vorzugsweise geht man daher so vor, daß man in einem Destillationsschritt unter thermisch schonenden Bedingungen mittels eines Dünnschichtverdampfers zunächst das niedrigsiedende Lösungsmittel und dann die Perfluorethercarbonsäuren von den festen oder hochsiedenden Verunreinigungen abtrennt. Anschließend werden die Perfluorethercarbonsäuren durch fraktionierte Vakuumdestillation voneinander getrennt und ohne Zersetzung in fluoridfreier Form erhalten.

Beispiele

Die nachstehend genannten Prozentzahlen sind Gewichtsprozente, sofern nichts anderes erwähnt oder ersichtlich ist. 1,1,2-Trichlor-1,2,2-trifluorethan wird kurz als Trichlortrifluorethan bezeichnet.

Vergleichsbeispiel 1

In einem 1l-Polyethylengefäß mit Rührer, Tropftrichter, Rückflußkühler und Innenthermometer wurden 200 g Wasser (11,1 Mol) vorgelegt und mit einem Kühlbad aus Eis/Wasser auf 10 - 15 °C gekühlt. Unter starkem Rühren wurden 498 g (1,0 Mol) $C_3F_7$-O-($C_3F_6$O)-CF($CF_3$)-COF zugetropft. Die beiden entstandenen Phasen wurden getrennt. Die organische Phase (460 g) enthielt 90,5 % $C_3F_7$-O-($C_3F_6$O)-CF($CF_3$)-COOH (Ausbeute 83,9 %), 9,4 % Wasser und 1370 ppm Fluorid. V4A-Stahl wurde von dieser organischen Phase korrodiert. Dies zeigt, daß bei einer Hydrolyse ohne organisches Lösungsmittel die gebildete Perfluorethercarbonsäure einen sehr hohen Fluoridgehalt hat.

Beispiel 1

In einem 1l-Polyethylengefäß mit Rührer, Tropftrichter, Rückflußkühler und Innenthermometer wurden 800 g Wasser (44,4 Mol) vorgelegt und unter Rühren auf 10 - 15 °C gekühlt. Eine Lösung von 2000 g $C_3F_7$-O-($C_3F_6$O)-CF($CF_3$)-COF (4,0 Mol) in 2000 g Trichlortrifluorethan wurde während 1 h zugetropft, wobei die Innentemperatur nicht über 25 °C anstieg. Nach beendeter Zugabe wurde 5 min nachgerührt; dann wurden die entstandenen beiden Phasen getrennt. Aus der organischen Phase wurden nach Abziehen des Lösungsmittels im Vakuum bei 25 °C 2035 g $C_3F_7$-O-($C_3F_6$O)-CF($CF_3$)-COOH mit einem Wassergehalt von 3,5 % und einem Fluoridgehalt von 30 ppm erhalten. Die Ausbeute betrug 98,5 %. Ein Korrosionstest ergab keinen Abtrag an V4A-Stahl.

Beispiel 2

Analog zu Beispiel 1 wurden 2000 g $C_3F_7$-O-($C_3F_6$O)$_2$-CF($CF_3$)-COF (3,0 Mol) in 2000 g Trichlortrifluorethan mit 600 g Wasser (33,3 Mol) umgesetzt. Es wurden 2010 g $C_3F_7$-O-($C_3F_6$O)$_2$-CF($CF_3$)-COOH mit einem Wassergehalt von 1,2 % und einem Fluoridgehalt von 15 ppm erhalten. Die Ausbeute betrug 99,6 %.

Beispiel 3

Analog zu Beispiel 1 wurden 2000 g $C_3F_7$-O-CF($CF_3$)-COF (6,0 Mol) in 2000 g Trichlortrifluorethan mit 1200 g Wasser (66,6 Mol) umgesetzt. Es wurden 2082 g $C_3F_7$-O-CF($CF_3$)-COOH mit einem Wassergehalt von 5,5 % und einem Fluoridgehalt von 190 ppm erhalten. Nach Destillation bei Normaldruck wurden 1942 g $C_3F_7$-O-CF($CF_3$)-COOH mit einem Siedepunkt von 143 °C, einem Wassergehalt < 0,3 % und einem Fluoridgehalt von 18 ppm erhalten. Die Ausbeute betrug 97,1 %.

Beispiel 4

In einem 100 ml-Polyethylengefäß wurden 30 g $C_3F_7$-O-($C_3F_6$O)-CF($CF_3$)-COF (60 mMol) in 30 g Perfluor-2-methylpentan gelöst. Unter intensivem Rühren wurden 11 g Wasser (0,61 Mol) während 5 min. zugegeben. Anschließende Phasentrennung ergab 30 g organische Phase, die 29 g $C_3F_7$-O-($C_3F_6$O)-CF-($CF_3$)- COOH, 1 g Wasser und 65 ppm Fluorid enthielt.

Beispiel 5

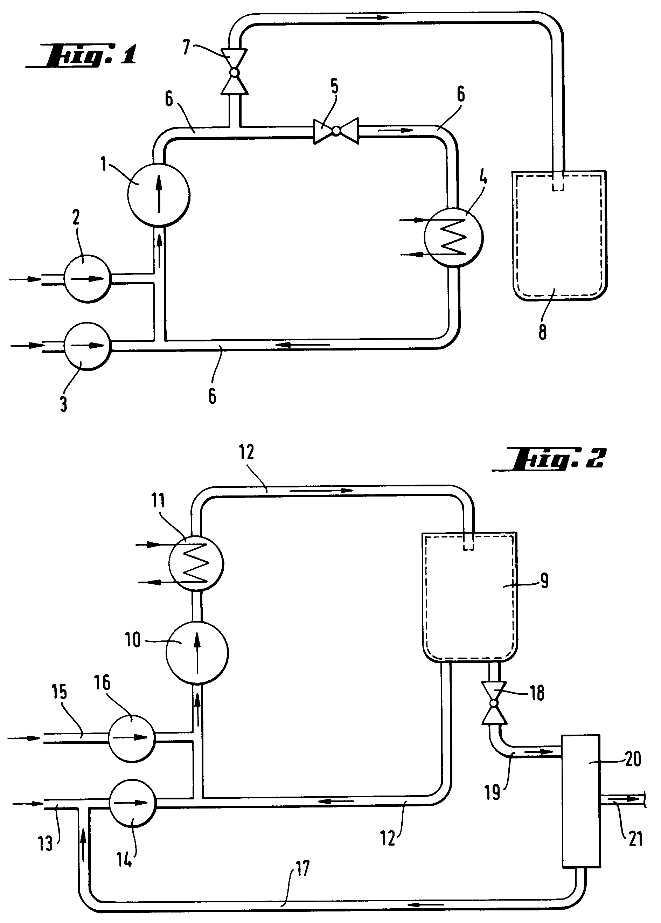

Als Apparatur wurde ein Loop-Reaktor (Fig. 1) benutzt, der aus einer Kreiselpumpe (1) (Fördermenge 60 l/h) zum Vermischen der Reaktanten, aus zwei Dosierpumpen (2) und (3), einem Wasserkühler (4) (Länge 20 cm) und einem regelbaren Ventil (5) bestand, die durch Kunststoffschläuche (6) (Durchmesser 0,8 cm) ringförmig miteinander und durch ein Entnahmeventil (7) mit einem Trenngefäß (8) verbunden waren. Das Reaktionsvolumen des Loopreaktors betrug 200 ml. Während 15 min. wurden 1500 g $C_3F_7$-O-($C_3F_6$O)-CF($CF_3$)-COF (3,0 Mol), verdünnt mit 1500 g Trichlortrifluorethan, und 580 g Wasser (32,2 Mol) über die Dosierpumpen (2) und (3) in die Apparatur gepumpt. Das Produkt wurde kontinuierlich über das Entnahmeventil (7) in das Trenngefäß (8) entnommen und dort in wäßrige und organische Phase getrennt. Als organische Phase wurden 1480 g $C_3F_7$-O-($C_3F_6$O)-CF($CF_3$)-COOH mit einem Wassergehalt von 1 % und einem Fluoridgehalt von 70 ppm erhalten. Die Ausbeute betrug 98,1 %

Beispiel 6

250 g $C_3F_7$-O-($C_3F_6$O)-CF($CF_3$)-COF (0,5 Mol) in 200 g Trichlortrifluorethan und 100 g Wasser (5,5 Mol) wurden während 15 min. mit Hilfe zweier Membranpumpen gleichzeitig durch ein 30 cm langes Kunststoffrohr (Innendurchmesser 18 mm, Volumen 76 $cm^3$) gepumpt, das Strömungsmischelemente enthielt. Nach Phasentrennung wurden 240 g $C_3F_7$-O-($C_3F_6$O)-CF($CF_3$)-COOH mit vollständigem Umsatz erhalten.

Beispiel 7

360 g eines durch Oligomerisation von Hexafluorpropenoxid und anschließende erfindungsgemäße Hydrolyse erhaltenen Gemisches (organische Phase) aus Trichlortrifluorethan und Perfluorethercarbonsäuren der Formel $C_3F_7$-O-($C_3F_6$O)$_n$-CF($CF_3$)-COOH (Zusammensetzung: 8 % Carbonsäure mit n = 0; 28 % mit n = 1; 8,7 % mit n = 2; 54 % Trichlortrifluorethan) mit einem Fluoridgehalt von 440 ppm und einem Wassergehalt von 3,5 % wurden zur Fluoridextraktion in einem Rührgefäß 10 min. mit 120 g Wasser vermischt. Durch Phasentrennung wurden 348 g organische Phase mit einem Fluoridgehalt von 77 ppm erhalten. Diese wurde über einen Dünnschichtverdampfer (Glas, Stahlwischer; Heiztemperatur 80 °C, Druck 1008 mbar) in 179 g Destillat (Trichlortrifluorethan) und 162 g Sumpf getrennt. Der Sumpf wurde bei 130 °C und 10 mbar über denselben Dünnschichtverdampfer getrennt in 2,2 g festen Rückstand, 2,9 g restliches Trichlortrifluorethan (in der Kühlfalle) und 154 g Destillat. Das Destillat wurde durch fraktionierte Vakuumdestillation an einer Füllkörperkolonne in 25 g $C_3F_7$-O-CF($CF_3$)-COOH (Siedepunkt 65 °C/40 mbar; Reinheit 99,1 %, Fluoridgehalt 14 ppm), 94 g $C_3F_7$-O-($C_3F_6$O)-CF($CF_3$)-COOH (Siedepunkt 87 °C/20 mbar; Reinheit 99,6 %, Fluoridgehalt 4 ppm) und 27 g $C_3F_7$-O-($C_3F_6$O)$_2$-CF($CF_3$)-COOH (Siedepunkt 129 °C/20 mbar; Reinheit 99,4 %, Fluoridgehalt 1,5 ppm) getrennt.

Beispiel 8

350 g $C_3F_7$-O-($C_3F_6$O)$_3$-CF($CF_3$)-COF (42,1 mMol) wurden mit 350 g Trichlortrifluorethan verdünnt und in einem Polyethylengefäß unter Rühren mit 84 g Wasser (4,7 Mol) versetzt. Nach Phasentrennung und Abziehen des Lösungsmittels bei 25 °C im Vakuum wurden 332 g $C_3F_7$-O-($C_3F_6$O)$_3$-CF($CF_3$)-COOH erhalten.

Beispiel 9

In einem Umpump-Reaktor (Fig. 2), der aus einem 600 l-Polyethylengefäß (9) mit Kühlmantel, Kreiselpumpe (10) ($3m^3/h$), Hastelloy-Kühler (11) und Ringleitung (12) wurden 60 kg Extraktionswasser aus einem früheren Extraktionsansatz über Leitung (13) und Dosierpumpe (14) vorgelegt und im Kreislauf gepumpt. 160 kg eines Gemisches oligomerer Perfluoretheracylfluoride der Formel $C_3F_7$-O-$(C_3F_6O)_n$-CF($CF_3$)-COF (n gleich 0, 1, und 2), das durch Oligomerisation von HFPO hergestellt worden war, wurden mit 155 kg Trichlortrifluorethan verdünnt und über Leitung (15), Dosierpumpe (16) und Ringleitung (12) in das Reaktionsgefäß (9) gepumpt (ca. 110 kg/h). Gleichzeitig wurden über Leitung (17) 60 kg Extraktionswasser (20 kg/h) zugepumpt. 30 min nach beendeter Zugabe wurde die Pumpe (10) des Kreislaufs abgeschaltet und die entstandenen zwei Phasen im Gefäß (9) getrennt. Die organische Phase wurde über Ventil (18) und Leitung (19) in eine Schwingbodenkolonne (20) aus Glas (Länge 3 m, Innendurchmesser 50 mm) überführt und dort mit 60 kg Wasser extrahiert, um den Fluoridgehalt zu verringern. Dabei wurden über Leitung (21) 320 kg Perfluorethercarbonsäurelösung (organische Phase) mit einem Wassergehalt von 3,5 % und einem Fluoridgehalt von 97 ppm erhalten. Mit 1l dieser Lösung wurden Werkstoffprüfungen an V4A-, Hastelloy C4- und Hastelloy-C22-Stahl über mehrere Tage in der Hitze durchgeführt. Alle Werkstoffe waren gegenüber der Lösung beständig, so daß deren weitere Aufarbeitung durch Destillation in einer gebräuchlichen Stahlapparatur durchgeführt werden konnte. Die Fluoridgehalte der destillierten Säuren betrugen 20 ppm ($C_3F_7$-O-CF($CF_3$)-COOH), 7 ppm ($C_3F_7$-O-($C_3F_6$O)-CF($CF_3$)-COOH) und 4 ppm ($C_3F_7$-O-$(C_3F_6O)_2$-CF($CF_3$)-COOH).

**Patentansprüche**

**1.** Verfahren zur Herstellung von Perfluorethercarbonsäuren der Formel (I) $R_f$-COOH aus Perfluoretheracylfluoriden der Formel (II):

(II) $R_f$-COF

mit $R_f$ gleich $C_mF_{2m+1}$-O-$(C_3F_6O)_n$-CF($CF_3$)-
worin m = 1 - 8 und n = 0 - 8 ist,

dadurch gekennzeichnet, daß man die Acylfluoride (II) mit der 0,5- bis 5-fachen Gewichtsmenge eines fluorierten inerten Lösungsmittels verdünnt und dann mit 2 - 15 Mol Wasser pro Mol Acylfluorid intensiv vermischt und die nach Phasentrennung erhaltene organische Phase destillativ vom Lösungsmittel befreit.

**2.** Verfahren nach Anspruch 1, dadurch gekennzeichnet, daß man als Lösungsmittel einen Chlorfluorkohlenwasserstoff der Formel $C_2Cl_3F_3$ oder $C_3Cl_2F_5H$ einsetzt.

**3.** Verfahren nach Anspruch 1, dadurch gekennzeichnet, daß man als Lösungsmittel einen fluorierten Ether oder Polyether der Formel

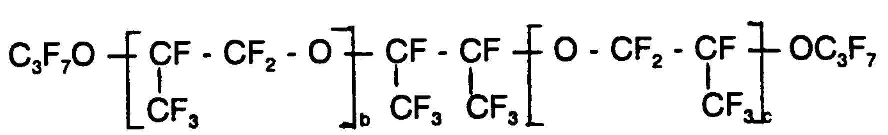

mit b = 0 - 4 und c = 0 - 4 oder

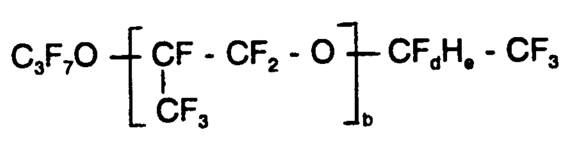

mit b = 0 - 4 und d = 1, e = 1 oder d = 2, e = 0 einsetzt.

**4.** Verfahren nach Anspruch 1, dadurch gekennzeichnet, daß man als Lösungsmittel ein Perfluoralkan der

Formel $C_aF_{2a+2}$ mit a = 5 - 12 einsetzt.

**5.** Verfahren nach Anspruch 1, dadurch gekennzeichnet, daß man als Lösungsmittel 1,1,2-Trichlor-1,2,2-trifluorethan, Perfluorhexan, Perfluorpropylethylether, Perfluorpropyltetrafluorethylether oder 1-H-Perfluorhexan einsetzt.

**6.** Verfahren nach Anspruch 1, dadurch gekennzeichnet, daß man als Lösungsmittel 1,1,2-Trichlor-1,2,2-trifluorethan einsetzt.

**7.** Verfahren nach einem der Ansprüche 1 bis 6, dadurch gekennzeichnet, daß man die nach der Hydrolyse erhaltene organische Phase mit Wasser extrahiert, bevor man sie destillativ vom Lösungsmittel befreit.

**8.** Verfahren nach einem der Ansprüche 1 bis 6, dadurch gekennzeichnet, daß man ein Gemisch von Perfluoretheracylfluoriden der Formel (II) mit m = 3 und n = 0, 1, 2 oder 3 einsetzt, die nach der Hydrolyse erhaltene organische Phase mit Wasser extrahiert und dann durch mehrstufige fraktionierte Destillation unter thermisch schonenden Bedingungen das Lösungsmittel entfernt und die erhaltenen Perfluorethercarbonsäuren der Formel (I) mit m = 3 und n = 0, 1, 2 oder 3 voneinander trennt.

**9.** Verfahren nach einem der Ansprüche 1 bis 8, dadurch gekennzeichnet, daß man die Hydrolyse kontinuierlich in einem Loop-Reaktor durchführt.

Fig. 1

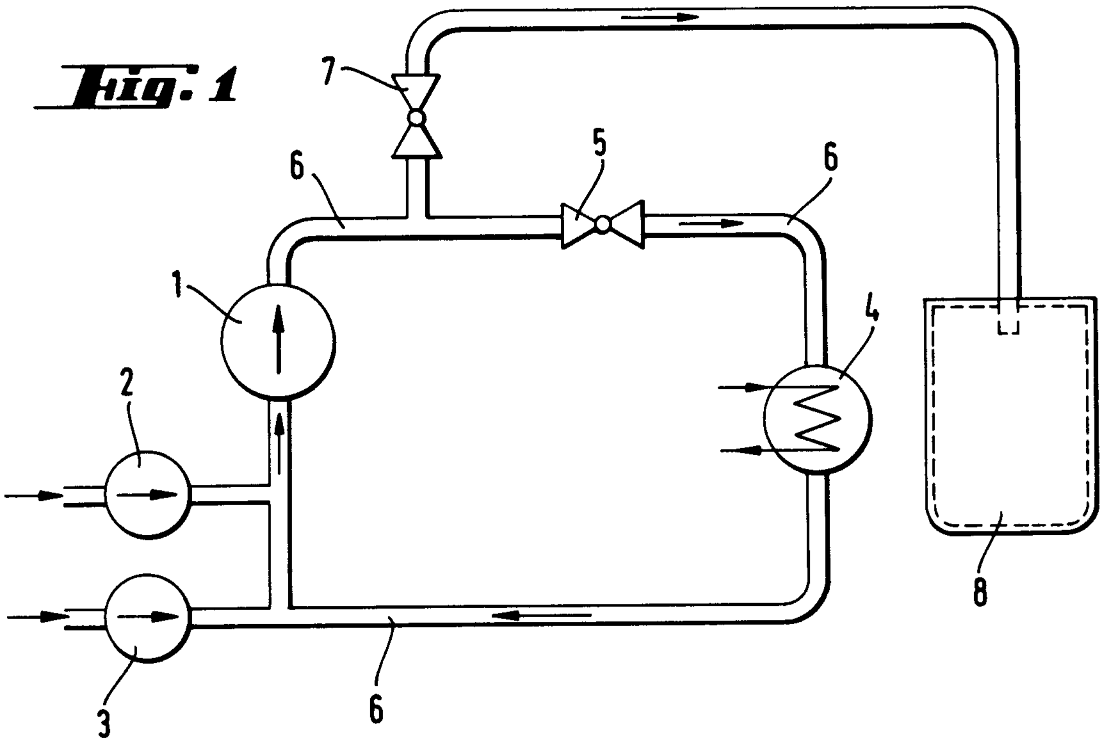


Fig. 2